# EUROPEAN PATENT SPECIFICATION

(11) **EP 3 711 758 B1**
(45) Date of publication and mention of the grant of the patent: **27.03.2024**
(21) Application number: 18878222.1
(22) Date of filing: 13.11.2018
(51) Int. Cl.: A61K 31/41, A61K 31/16, A61P 25/08, A61P 25/10

(54) **USE OF CARBAMATE COMPOUND FOR PREVENTING, ALLEVIATING OR TREATING ABSENCE SEIZURE OR EPILEPSY SHOWING ABSENCE SEIZURE**
VERWENDUNG EINER CARBAMATVERBINDUNG ZUR VORBEUGUNG, LINDERUNG ODER BEHANDLUNG VON ABSENCE-ANFÄLLEN ODER EPILEPSIE MIT ABSENCE-ANFÄLLEN
UTILISATION D'UN COMPOSÉ DE CARBAMATE POUR PRÉVENIR, SOULAGER OU TRAITER LES CRISES D'ABSENCE OU LES CRISES D'ABSENCE ÉPILEPTIQUES

(30) Priority: 14.11.2017 KR 20170151248
(43) Date of publication of application: 23.09.2020
(73) Proprietor: SK Biopharmaceuticals Co., Ltd., Seongnam-si, Gyeonggi-do 13494 (KR)
(72) Inventor: SHIN, Hye Won, Seongnam-si Gyeonggi-do 13494 (KR)
(74) Representative: Vossius & Partner Patentanwälte Rechtsanwälte mbB
(86) International application number: PCT/KR2018/013761
(87) International publication number: WO 2019/098628

(56) References cited:
- WO-A1-2006/112685
- KR-A- 20080 005 437
- KR-A- 20100 137 389
- KR-A- 20120 087 124
- MEIR BIALER ET AL: "Progress report on new antiepileptic drugs: A summary of the Eleventh Eilat Conference (EILAT XI)", EPILEPSY RESEARCH, vol. 103, no. 1, 1 January 2013 (2013-01-01), pages 2-30, XP055691206, NL ISSN: 0920-1211, DOI: 10.1016/j.eplepsyres.2012.10.001
- GOLYALA AMBICA ET AL: "Drug development for refractory epilepsy: The past 25 years and beyond", SEIZURE, BAILLIERE TINDALL, LONDON, GB, vol. 44, 6 December 2016 (2016-12-06), pages 147-156, XP029901803, ISSN: 1059-1311, DOI: 10.1016/J.SEIZURE.2016.11.022
- BIALER, M. et al.: "Progress Report on new Antiepileptic Drugs: A Summary of the Twelfth Eilat Conference (EILAT XII", Epilepsy Research, vol. 1, no. 11, 2015, pages 85-141, XP055608941,
- GOLYALA, A. et al.: "Drug Development for Refractory Epilepsy: The Past 25 Years and Beyond", Seizure, vol. 44, January 2017 (2017-01), pages 147-156, XP029901803, DOI: doi:10.1016/j.seizure.2016.11.022
- LÖSCHER WOLFGANG: "Fit for purpose application of currently existing animal models in the discovery of novel epilepsy therapies", EPILEPSY RESEARCH, ELSEVIER SCIENCE PUBLISHERS , AMSTERDAM, NL, vol. 126, 1 August 2016 (2016-08-01), pages 157-184, XP029709265, ISSN: 0920-1211, DOI: 10.1016/J.EPLEPSYRES.2016.05.016

## Description

### [Technical field]

The present invention relates to a carbamate compound of the following Formula 1, or a pharmaceutically acceptable salt, solvate or hydrate thereof for use in preventing, alleviating or treating absence seizure or epilepsy showing absence seizure: 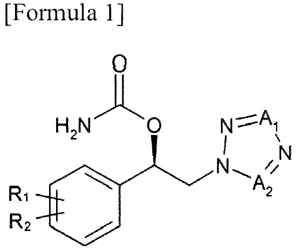 wherein,
R₁, R₂, A₁ and A₂ are as defined herein.

### [Background art]

All symptoms caused by transient and irregular abnormal excitation of nerve cells as a whole are called seizures. Epilepsy refers to a group of diseases in which chronic seizures occur even when there are no abnormalities in the body that can cause seizures, such as a special causative factor, for example, electrolyte imbalance, acid-base abnormality, uremia, alcohol withdrawal, severe sleep deprivation, etc. It is known that continuous treatment with medication is necessary in cases of two or more seizures.

An epilepsy seizure is classified based on clinical symptoms and electroencephalography (EEG) findings. According to this classification, epilepsy seizures are largely divided into partial seizures and generalized seizures. Partial seizures refer to hyperexcitatory seizures of nerve cells that start in a part of the cerebral cortex, and generalized seizures refer to seizures that start in a wide range of both the cerebral hemispheres. The types of generalized seizures include generalized tonic-clonic seizures, absence seizures (small seizures), myoclonic seizures and atonic seizures. The types of partial seizures include simple partial seizures, complex partial seizures and secondary generalized seizures resulting from partial seizures (Source: Korean Epilepsy Society).

Out of these, absence seizures (small seizures) are characterized by the symptoms of suddenly stopping the action and staring blankly or dropping the head for about 5 to 10 seconds. Occasionally, slight shaking of the eyes or around the mouth can be observed. These symptoms usually appear when breathing hard (Source: Korean Epilepsy Society). The two most characteristic features of absence seizures are the clinical symptom of impairment of consciousness and the electroencephalography (EEG) showing spike-and-wave discharges (SWD). The types of epilepsy with symptoms of absence seizures include child absence epilepsy, epilepsy with myoclonic absence, juvenile absence epilepsy and juvenile myoclonic epilepsy.

Absence seizures can be divided into a typical absence and an atypical absence. In case of typical absence, seizures usually end within 2 to 10 seconds and rarely last longer than 30 seconds. Therefore, patients themselves, much less others, sometimes do not know whether they have had seizures. After a seizure, patients return to the action or situation they were doing just before the seizure without realizing that they had a seizure. Atypical absence includes symptoms of loss of consciousness, staring with wide-open eyes, blinking of the eyelids, licking the lips, chewing behavior or touching fingers. The frequency of these symptoms may decrease before and after puberty, but most tend to develop into a grand mal seizure.

There are limited types of treatments for absence seizures. Valproic acid or ethosuximide is mainly used, and lamotrigine can also be used, but is known to be less effective (Glauser et al. (2010) "Ethosuximide, Valproic Acid, and Lamotrigine in Childhood Absence Epilepsy," New England Journal of Medicine, 362 (9): 790-9). However, other antiepileptic drugs such as carbamazepine, vigabatrin, tiagabine, oxcarbazepine, phenytoin, phenobarbital, gabapentin and pregabalin can rather exacerbate the absence seizures, and thus are contraindicated in patients with absence seizures ("NICE Guidelines" Retrieved November 3, 2014; Knake et al., (August 8, 1999) "Tiagabine-induced absence status in idiopathic generalized epilepsy," European Journal of Epilepsy 8 (5): 314-317).

WO2006/112685A1 discloses compounds of Formula IX. The compounds of this document are tested in pentylenetetrazol (PTZ)-induced clonic seizure rat model. The document further discloses that the compounds that antagonize the effects of subcutaneous PTZ-induced clonic seizures are known to elevate the seizure threshold, hence are generally useful in preventing absence seizures.

Carboxamide dibenzazepine-based drugs are used to treat partial seizures, but drugs of this class are known to exacerbate general seizures, especially absence seizures.

### [Disclosure of the Invention]

The invention is defined by the appended claims. Any subject-matter falling outside the scope of the claims is provided for information purposes, only. The references to methods of treatment in this description are to be interpreted as references to the compounds, pharmaceutical compositions and medicaments of the present invention for use in a method for treatment of the human (or animal) body by therapy (or for diagnosis).

### [Problem to be solved]

The present invention is intended to provide a method for the prevention, alleviation or treatment of absence seizure or epilepsy showing absence seizure.

The present invention is also intended to provide the use of a carbamate compound of the following Formula 1, or a pharmaceutically acceptable salt, solvate or hydrate thereof, for the prevention, alleviation or treatment of absence seizure or epilepsy showing absence seizure: 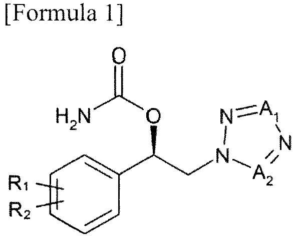 wherein,
R₁, R₂, A₁ and A₂ are as defined herein.

### [Technical Solution to the Problem]

The present invention provides a medicament for the prevention, alleviation or treatment of absence seizure or epilepsy showing absence seizure, comprising a therapeutically effective amount of a carbamate compound of the following Formula 1, or a pharmaceutically acceptable salt, solvate or hydrate thereof: 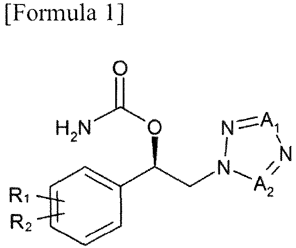 wherein,
R₁ and R₂ are each independently selected from the group consisting of hydrogen, halogen, C₁-C₈ alkyl, halo-C₁-C₈ alkyl, C₁-C₈ thioalkoxy and C₁-C₈ alkoxy; and
one of A₁ and A₂ is CH, and the other is N.

In addition, the present invention provides a pharmaceutical composition for the prevention, alleviation or treatment of absence seizure or epilepsy showing absence seizure, comprising a therapeutically effective amount of the carbamate compounds of the above Formula 1, or a pharmaceutically acceptable salt, solvate or hydrate thereof, and further one or more of a pharmaceutically acceptable carrier.

In addition, the present invention provides a method for preventing, alleviating or treating absence seizure or epilepsy showing absence seizure in a subject, comprising administering to the subject a therapeutically effective amount of the carbamate compounds of the above Formula 1, or a pharmaceutically acceptable salt, solvate or hydrate thereof.

In addition, the present invention provides the use of the carbamate compounds of the above Formula 1, or a pharmaceutically acceptable salt, solvate or hydrate thereof for the prevention, alleviation or treatment of absence seizure or epilepsy showing absence seizure.

In one embodiment of the present invention, in the above Formula 1, R₁ and R₂ are each independently selected from the group consisting of hydrogen, halogen and C₁-C₈ alkyl.

In one embodiment of the present invention, the halo C₁-C₈ alkyl is perfluoroalkyl.

According to another embodiment of the present invention, the carbamate compound of the above Formula 1 is carbamic acid (R)-1-(2-chlorophenyl)-2-tetrazol-2-yl-ethyl ester of the following Formula 2:

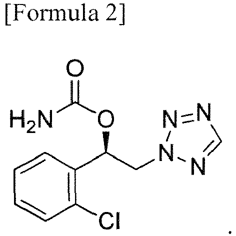

A person having ordinary skill in the art of synthesis of compounds could have easily prepared the carbamate compounds of the above Formulas 1 and 2 using known compounds or compounds which can be easily prepared therefrom. In particular, methods for preparing the compounds of the above Formula 1 are described in detail in PCT Publication Nos. WO 2006/112685 A1, WO 2010/150946 A1 and WO2011/046380 A2.

The compounds of the above Formula 1 can be chemically synthesized by any of the methods described in the above documents, but the methods are merely exemplary ones, and the order of the unit operation and the like may be selectively changed if necessary.

The carbamate compounds of the above Formula 1 can be used for the prevention, alleviation or treatment of absence seizure or epilepsy showing absence seizure.

According to one embodiment of the present invention, the absence seizures can be largely divided into typical absence and atypical absence. In case of typical absence, seizures usually end within 2 to 10 seconds and rarely last longer than 30 seconds. Therefore, patients themselves, much less others, sometimes do not know whether they have had seizures. After a seizure, patients return to the action or situation they were doing just before the seizure without realizing that they had a seizure. Atypical absence includes symptoms of loss of consciousness, staring with wide-open eyes, blinking of the eyelids, licking the lips, chewing behavior or touching fingers. The frequency of these symptoms may decrease before and after puberty, but most tend to develop into a grand mal seizure.

According to one embodiment of the present invention, epilepsy showing absence seizures can be one or more selected from child absence epilepsy, epilepsy with myoclonic absence, juvenile absence epilepsy, juvenile myoclonic epilepsy, Jeavons syndrome (eyelid myoclonia with absence), genetic generalized epilepsy with phantom absence and Lennox-Gastaut syndrome.

According to one embodiment of the present invention, absence seizures can be a typical absence or an atypical absence. Absence seizures can also include all seizures having spike-and-wave discharges (SWD) as a characteristic of EEG.

The carbamate compounds of the above Formula 1 may be applied to absence seizures or epilepsy showing absence seizures through their ability to inhibit spike-and-wave discharges (SWD).

The efficacy of the carbamate compounds of the above Formula 1 for absence seizures or epilepsy showing absence seizures can be confirmed using a known model. For example, the GAERS (Genetic Absence Epilepsy Rat from Strasbourg) model is a type of rat that exhibits spontaneous spike-and-wave discharges (SWD) and shows the behavior of absence seizures due to its genetic characteristics, and thus it is an experimental animal model that is being used as a means to verify the efficacy of an agent for the treatment of absence seizures (Depaulis et al., The genetic absence epilepsy rat from Strasbourg as a model to decipher the neuronal and network mechanisms of generalized idiopathic epilepsies. J Neurosci Methods, 2016 Feb 15;260:159-74).

In this disease model rat, SWD is characteristically observed through cerebral cortical EEG recording and lasts for about 17 to 25 seconds at a time and occurs about 45 to 60 times per hour. This SWD is also associated with the suspension of activity, a behavioral feature of absence seizures. It is known that SWD in the GAERS model rats is also inhibited by valproate, ethosuximide and the like, similar in human patients, and it is known that a large number of drugs have no inhibitory effect or some drugs rather worsen SWD (Dedeurwaerdere et al., Fluctuating and constant valproate administration gives equivalent seizure control in rats with genetic and acquired epilepsy. Seizure. 2011 Jan;20(1):72-9; Nehlig et al., Cerebral energy metabolism in rats with genetic absence epilepsy is not correlated with the pharmacological increase or suppression of spike-wave discharges. Brain Res. 1993 Jul 30;618(1):1-8; Wallengren et al., Aggravation of absence seizures by carbamazepine in a genetic rat model does not induce neuronal c-Fos activation. Clin Neuropharmacol. 2005 Mar-Apr;28(2):60-5; Gurbanova et al., Effect of systemic and intracortical administration of phenytoin in two genetic models of absence epilepsy. Br J Pharmacol. 2006 Aug;148(8):1076-82; Vartanian et al., Activity profile of pregabalin in rodent models of epilepsy and ataxia. Epilepsy Res. 2006 Mar;68(3):189-205).

Therefore, this disease model is considered to be a useful model in predicting the effect on absence seizures (Klitgaard et al., Use of epileptic animals for adverse effect testing. Epilepsy Res. 2002 Jun;50(1-2):55-65; van Luijtelaar EL, Drinkenburg WH, van Rijn CM, Coenen AM. Rat models of genetic absence epilepsy: what do EEG spike-wave discharges tell us about drug effects? Methods Find Exp Clin Pharmacol. 2002;24 Suppl D:65-70).

The dosage of the carbamate compounds of Formula 1 for the prevention, alleviation or treatment of the above diseases may typically vary depending on the severity of the disease, the body weight and the metabolic status of the subject. A "therapeutically effective amount" for an individual patient refers to an amount of the active compound sufficient to achieve the above pharmacological effect, i.e., the therapeutic effect as described above. The therapeutically effective amount of the compounds of Formula 1 is 50 to 500 mg, 50 to 400 mg, 50 to 300 mg, 100 to 400 mg, 100 to 300 mg, 50 to 200 mg, or 100 to 200 mg, based on the free form and once-daily administration to humans.

The compounds of the present invention may be administered by any conventional method used for administration of a therapeutic agent, such as oral, parenteral, intravenous, intramuscular, subcutaneous or rectal administration.

The medicament or pharmaceutical composition according to one embodiment of the present invention may comprise a therapeutically effective amount of a compound selected from the group consisting of the carbamate compounds of the present invention, their pharmaceutically acceptable salts, solvates, hydrates and combinations thereof.

Examples of the pharmaceutically acceptable salts of the carbamate compounds of the above Formula 1 include independently, acetate, benzenesulfonate, benzoate, bitartrate, calcium acetate, camsylate, carbonate, citrate, edetate, edisylate, estolate, esylate, fumarate, gluceptate, gluconate, glutamate, glycoloyl arsanilate, hexylresorcinate, hydravamine, hydrobromide, hydrochloride, hydrogencarbonate, hydroxynaphthoate, iodide, isethionate, lactate, lactobionate, malate, maleate, mandelate, mesylate, methylnitrate, methyl sulfate, mucate, napsylate, nitrate, pamoate (embonate), pantothenate, phosphate/diphosphate, polygalacturonate, salicylate, stearate, subacetate, succinate or hemi-succinate, sulfate or hemi-sulfate, tannate, tartrate, oxalate or hemi-tartrate, teoclate, triethiodide, benzathine, chloroprocaine, choline, diethanolamine, ethylenediamine, meglumine, procaine, aluminum, ammonium, tetramethylammonium, calcium, lithium, magnesium, potassium, sodium and zinc.

The medicament or pharmaceutical composition according to one embodiment of the present invention may be administered orally or parenterally. The parenteral administration may include intravenous injection, subcutaneous injection, intramuscular injection, intraperitoneal injection, endothelial administration, topical administration, intranasal administration, intravaginal administration, intrapulmonary administration, rectal administration and the like. In the case of oral administration, the pharmaceutical composition according to one embodiment of the present invention may be formulated as a plain tablet (uncoated tablet) or such that the active agent is coated or it is protected against degradation in the stomach. In addition, the composition can be administered by any device capable of transferring the active substance to a target cell. The route of administration may vary depending upon the general condition and age of the subject to be treated, the nature of the treatment condition and the active ingredient selected.

A suitable dosage of the medicament or pharmaceutical composition according to one embodiment of the present invention may vary depending on factors such as the formulation method, administration method, age, body weight and gender of patients, pathological condition, diet, administration time, administration route, excretion rate and reaction sensitivity, and doctors having ordinary skill can easily determine and prescribe dosages that are effective for the desired treatment or prophylaxis. The pharmaceutical composition according to one embodiment may be administered in one or more doses, for example, one to four times per day. The pharmaceutical composition according to one embodiment may contain the compounds of Formula 1 in the amount of 50 to 500 mg, 50 to 400 mg, 50 to 300 mg, 100 to 400 mg, 100 to 300 mg, 50 to 200 mg, or 100 to 200 mg, based on the free form.

The medicament or pharmaceutical composition according to one embodiment of the present invention may be formulated using a pharmaceutically acceptable carrier and/or excipient according to a method that a person having ordinary skill in the art could easily carry out, thereby to be prepared in a unit dose form or to be contained in a multi-dose container. The above formulation may be a solution in oil or an aqueous medium, a suspension or an emulsion (emulsified solution), an extract, a powder, granules, a tablet, or a capsule, and may further include a dispersing or stabilizing agent. In addition, the pharmaceutical composition may be administered in the form of suppositories, sprays, ointments, creams, gels, inhalants or skin patches. The pharmaceutical composition may also be prepared for mammalian administration, more preferably for human administration.

Pharmaceutically acceptable carriers may be solid or liquid, and may be one or more selected from fillers, antioxidants, buffers, bacteriostats, dispersants, adsorbents, surfactants, binders, preservatives, disintegrants, sweeteners, flavors, glidants, release-controlling agents, wetting agents, stabilizers, suspending agents, and lubricants. In addition, the pharmaceutically acceptable carriers may be selected from saline, sterile water, Ringer's solution, buffered saline, dextrose solution, maltodextrin solution, glycerol, ethanol and mixtures thereof.

In one embodiment, suitable fillers include, but are not limited to, sugar (e.g., dextrose, sucrose, maltose and lactose), starch (e.g., corn starch), sugar alcohol (e.g., mannitol, sorbitol, maltitol, erythritol and xylitol), starch hydrolysate (e.g., dextrin and maltodextrin), cellulose or cellulose derivatives (e.g., microcrystalline cellulose) or mixtures thereof.

In one embodiment, suitable binders include, but are not limited to, povidone, copovidone, methylcellulose, hydroxypropylmethylcellulose, hydroxypropylcellulose, hydroxyethylcellulose, gelatin, gum, sucrose, starch or mixtures thereof.

In one embodiment, suitable preservatives include, but are not limited to, benzoic acid, sodium benzoate, benzyl alcohol, butylated hydroxyanisole, butylated hydroxytoluene, chlorbutol, gallate, hydroxybenzoate, EDTA or mixtures thereof.

In one embodiment, suitable disintegrants include, but are not limited to, sodium starch glycolate, cross-linked polyvinylpyrrolidone, cross-linked carboxymethylcellulose, starch, microcrystalline cellulose or mixtures thereof.

In one embodiment, suitable sweeteners include, but are not limited to, sucralose, saccharin, sodium saccharin, potassium saccharin, calcium saccharin, acesulfame potassium or sodium cyclamate, mannitol, fructose, sucrose, maltose or mixtures thereof.

In one embodiment, suitable glidants include, but are not limited to, silica, colloidal silicon dioxide, talc and the like.

In one embodiment, suitable lubricants include, but are not limited to, long chain fatty acids and salts thereof, such as magnesium stearate and stearic acid, talc, glyceride wax or mixtures thereof.

As used herein, the terms "prevent," "preventing" and "prevention" refer to reducing or eliminating the likelihood of a disease.

As used herein, the terms "alleviate," "alleviating" and "alleviation" refer to ameliorating a disease and/or its accompanying symptoms altogether or in part.

As used herein, the terms "treat," "treating" and "treatment" refer to eliminating a disease and/or its accompanying symptoms altogether or in part.

As used herein, the term "subject" refers to an animal that is the object of therapy, observation or experiment, preferably a mammal (such as primates (e.g., a human), cattle, sheep, goats, horses, dogs, cats, rabbits, rats, mice, etc.), most preferably a human.

As used herein, the term "therapeutically effective amount" refers to the amount of active compound or pharmaceutical formulation that elicits a biological or medical response in the system, animal or human, including alleviation of the symptoms of the disease or disorder to be treated, wherein said amount is sought by a researcher, veterinarian, doctor (physician) or other clinician.

As used herein, the term "composition" encompasses a product that contains a specified amount of a particular ingredient and any product that results directly or indirectly from a combination of specified amounts of particular ingredients.

### [Effect of the Invention]

The medicament and the pharmaceutical composition according to the present invention can effectively treat and prevent absence seizure or epilepsy showing absence seizure.

### [Brief Description of the Drawings]

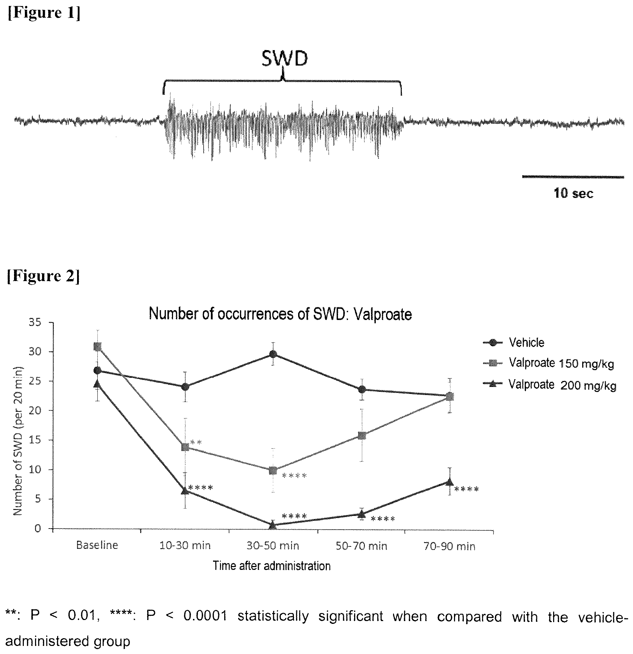

Figure 1 shows the results of electroencephalography (EGG) measurements observed under baseline conditions of the GAERS model used in the Examples. It can be seen that spontaneous SWD occurs.
Figure 2 shows the result of analyzing the effect of valproate used as a positive control on the number of occurrences of SWD generated in the GAERS model through electroencephalography (EEG) analysis. The efficacy at two doses of 150 mg/kg and 200 mg/kg was analyzed. The number of occurrences of SWD in the baseline period of 20 minutes immediately before drug administration and the number of occurrences of SWD in a total of 80 minutes from 10 minutes to 90 minutes after drug administration are shown.

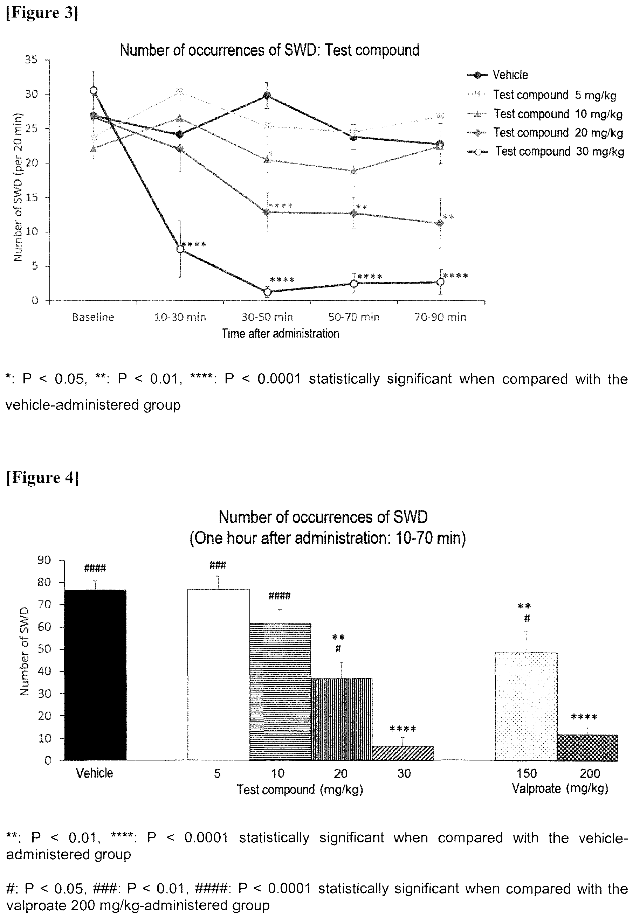

Figure 3 shows the results of analyzing the effect of the test compound on the number of occurrences of SWD generated in the GAERS model through EEG analysis. The efficacy at four doses of 5, 10, 20 and 30 mg/kg was analyzed. The number of occurrences of SWD in the baseline period of 20 minutes immediately before administration of the test compound and the number of occurrences of SWD in a total of 80 minutes from 10 minutes to 90 minutes after administration of the test compound are shown.
Figure 4 shows the number of occurrences of SWD for a total of 60 minutes from 10 to 70 minutes after the drug or test compound administration. The difference in effect between the vehicle administration group, the test compound administration group and the valproate administration group can be confirmed.

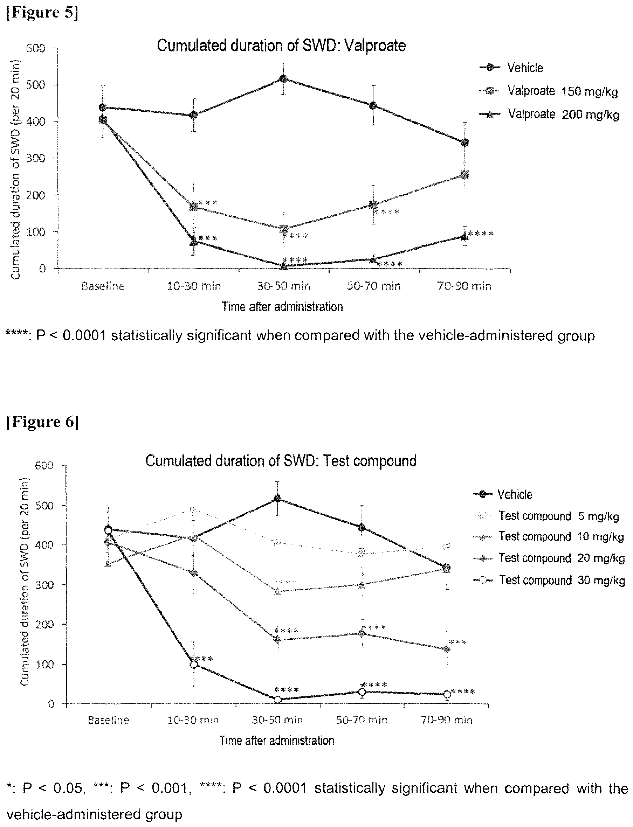

Figure 5 shows the results of analyzing the effect of valproate used as a positive control on the cumulated duration of SWD generated in the GAERS model over time through EEG analysis. The efficacy at two doses of 150 mg/kg and 200 mg/kg was analyzed. The cumulated duration of SWD in the baseline period of 20 minutes immediately before drug administration and the cumulated duration of SWD in 20-minute unit intervals from 10 minutes to 90 minutes after drug administration are shown.
Figure 6 shows the results of analyzing the effect of the test compound on the cumulated duration of SWD generated in the GAERS model over time through EEG analysis. The efficacy at four doses of 5, 10, 20 and 30 mg/kg was analyzed. The cumulated duration of SWD in the baseline period of 20 minutes immediately before administration of the test compound and the cumulated duration of SWD in 20-minute unit intervals from 10 minutes to 90 minutes after administration of the test compound are shown.

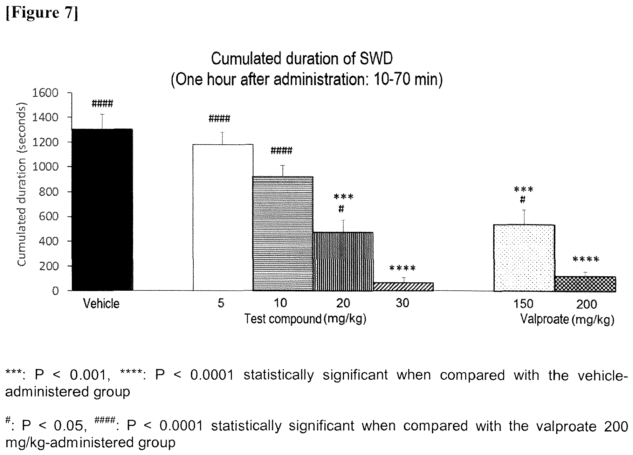

Figure 7 shows the cumulated duration of SWD in 20-minute unit intervals of a total of 60 minutes from 10 to 70 minutes after the drug or test compound administration. The difference in effect between the vehicle administration group, the test compound administration group and the valproate administration group can be confirmed.

### [Specific embodiments to carry out the invention]

Hereinafter, the present invention will be explained in more detail through working examples. However, the following working examples are only intended to illustrate one or more embodiments and are not intended to limit the scope of the invention.

### Preparation Example: Synthesis of carbamic acid (R)-1-(2-chlorophenyl)-2-tetrazol-2-yl-ethyl ester

Carbamic acid (R)-1-(2-chlorophenyl)-2-tetrazol-2-yl-ethyl ester (test compound) was prepared according to the method described in Preparation Example 50 of PCT Publication No. WO 2010/150946.

### Example: Efficacy test of prevention and treatment of absence seizures using GAERS (Genetic Absence Epilepsy Rat from Strasbourg) models

The GAERS (Genetic Absence Epilepsy Rat from Strasbourg) model is a type of rat that exhibits spontaneous SWD due to genetic effects. The GAERS model possesses typical behavioral, electrophysiological and pharmacological characteristics of absence seizure, and thus it has been used as a disease model for absence seizure for about 20 years (Depaulis et al., The genetic absence epilepsy rat from Strasbourg as a model to decipher the neuronal and network mechanisms of generalized idiopathic epilepsies. J Neurosci Methods. 2016 Feb 15;260:159-74).

### Experimental animals

Twelve 5-week-old GAERS model rats were purchased from the Grenoble Institute of Neurosciences, France. After purchase, the rats were bred at a temperature of 21±2°C under conditions of free access to food and water, and controlled light (illuminated from 8 am to 8 pm), and the condition was maintained for 2 months prior to surgery.

### Electrode implantation for EEG measurement

EEG electrodes were implanted into twelve 3-month-old GAERS rats. Stereotaxic implantation was performed under anesthesia with isoflurane (2% concentration in oxygen). The rats were fixed to the stereotaxic frame and surgery was performed while observing body temperature. An ophthalmic gel was used to prevent drying of the cornea. After cutting the hair of the incision site, it was disinfected with povidone, and after incision, four holes were drilled using a drill at the frontal cortical and parietal cortical positions of both hemispheres of the open skull. Monopolar electrodes were placed in these holes and then connected to a female connector fixed to the skull for EEG recording. The incision site was then sutured. After surgery, buprenorphine was injected subcutaneously at a dose of 0.1 mg/kg, and the anesthetic was removed and observed until recovery of consciousness. After that, the rats were kept in a cage for recovery for a week.

### Animal screening for drug efficacy test

One week after the electrode implantation, preliminary EEG measurements of 1 hour were performed on all 12 animals, and 10 animals showing enough signal-to-noise suitable for detecting generation of SWD were selected.

### Drug administration

Test compound and positive control drug were prepared immediately before administration. Carbamic acid (R)-1-(2-chlorophenyl)-2-tetrazol-2-yl-ethyl ester prepared in the above preparation example was used as the test compound which was dissolved in 30% (volume/volume) PEG300 (81164-14, Sigma-Aldrich), and the positive control valproate (P4543, Sigma-Aldrich) was dissolved in 0.9% sterile saline (Cooper, France). Both drugs were administered intraperitoneally at a dose of 5 ml/kg. 30% PEG300 was used for vehicle administration as the negative control.

The test was done in a cross-over method, with a minimum interval of 71 hours between each administration for wash-out of the drug. In the first stage test, the following conditions were administered.
- Vehicle (30% PEG300)
- Valproate 200 mg/kg
- Test compound 5 mg/kg
- Test compound 10 mg/kg
- Test compound 20 mg/kg

In the second stage test, the following conditions were administered.
- Valproate 150 mg/kg
- Test compound 30 mg/kg

### EEG recording

For EEG recording, 10 GAERS rats were placed in a recording chamber and then connected to a wire for EEG recording. After an hour of habituation process, EEG recordings of free-moving animals were performed. As a baseline phase, 20 minutes of recording were performed before drug administration, and 90 minutes of recording were performed after drug administration, using SystemPlus Evolution (Micromed). During the recording, the animals were kept in a quiet wakefulness state.

### EEG data analysis

The generated SWD was analyzed using the recorded EEG data, and all data analysis was performed by blind method. SWD was analyzed for the 20-minute baseline phase and for a total of 80 minutes period from 10 to 90 minutes after drug administration. The 80 minutes were analyzed by dividing them into 20-minute intervals, and the number of occurrences of SWD and the cumulated SWD duration (the cumulated duration of SWD) were analyzed.

### Statistical analysis

Results were expressed as mean value ± standard error, and prism (graphpad) was used for statistical analysis. 2-way ANOVA was used for the number of occurrences of SWD and the cumulated duration of SWD over time. Bonferroni's paired comparison method was used for comparison with the baseline period, comparison with the vehicle-administered negative control and comparison with the valproate-administered positive control. Regarding the total effect for 1 hour after drug administration, 1-way ANOVA was used for repeated measurement values, and Bonferroni's paired comparison method was used for comparison with the vehicle-administered negative control and comparison with the valproate-administered positive control. The criterion for statistical significance was defined as p <0.05.

### Test results

In one out of the 10 GAERS rats used for EEG recording, the number of baseline SWDs generated was low, making it unsuitable for analysis, and the data of that one were excluded from final analysis.

### 1) Effect of test compound on the number of occurrences of SWD

The results of 2-way ANOVA analysis of the measurement values for each 20-minute unit interval are as follows.
- The vehicle administration group as the negative control did not show a difference compared to the baseline at any time point.
- The valproate administration group as the positive control showed an effect of reducing the number of occurrences of SWD by valproate administration. The 150 mg/kg administration group showed a statistically significant decrease in the number of occurrences of SWD compared to the vehicle administration group in the 10-30 min and 30-50 min intervals after administration. In addition, the 200 mg/kg administration group showed a significant decrease in the number of occurrences of SWD compared to the negative control in the entire time period (between 10 and 90 minutes) after administration.
- The test compound administration group at the doses of 10, 20 and 30 mg/kg showed a significant decrease in the number of occurrences of SWD compared to the vehicle administration group. The 10 mg/kg dose showed significance in the 30 to 50 min interval after administration, the 20 mg/kg dose showed significance in the 30 to 90 min interval after administration, and the 30 mg/kg dose showed significance in the entire period after administration. In terms of effect duration, the test compound showed a longer duration than valproate as the positive control.

Regarding the total effect for 1 hour after drug administration, the results of 1-way ANOVA analysis indicated that the test compound administration group at the doses of 20 and 30 mg/kg significantly reduced the number of occurrences of SWD compared to the vehicle administration group, and showed an effect similar to that of the valproate administration group at the doses of 150 and 200 mg/kg.

### 2) Effect of test compound on the cumulated duration of SWD

The results of 2-way ANOVA analysis of the cumulated values of SWD duration for each 20-minute unit interval are as follows.
- The vehicle administration group as the negative control did not show a difference compared to the baseline at any time point.
- The valproate administration group as the positive control showed a reduction effect of the cumulated duration of SWD by valproate administration. The 150 mg/kg administration group showed a significant decrease in 10-30 min, 30-50 min and 50-70 min intervals after administration, and the 200 mg/kg administration group showed a significant decrease compared to the negative control in the entire time period (between 10 and 90 minutes) after administration.
- The test compound administration group at the doses of 10, 20 and 30 mg/kg showed a significant decrease in the cumulated duration of SWD compared to the vehicle administration group. The 10 mg/kg dose showed a significant reduction effect in the 30-50 min and 50-70 min intervals after administration, the 20 mg/kg dose showed a significant reduction effect in the 30-90 min interval after administration, and the 30 mg/kg dose showed a significant reduction effect in the entire period after administration. In terms of effect duration, the test compound showed a longer duration than valproate as the positive control.

Regarding the total effect for 1 hour after drug administration, the results of 1-way ANOVA analysis indicated that the test compound administration group at the doses of 20 and 30 mg/kg significantly reduced the cumulated duration of SWD compared to the vehicle administration group, and showed an effect similar to that of the valproate administration group at the doses of 150 and 200 mg/kg.

## Claims

1. A carbamate compound of the following Formula 1, or a pharmaceutically acceptable salt, solvate or hydrate thereof for use in the prevention, alleviation or treatment of absence seizure or epilepsy showing absence seizure: 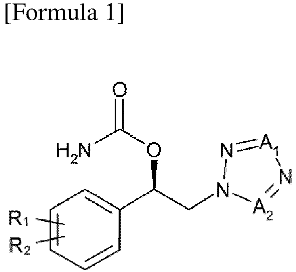 wherein,
R₁ and R₂ are each independently selected from the group consisting of hydrogen, halogen, C₁-C₈ alkyl, halo-C₁-C₈ alkyl, C₁-C₈ thioalkoxy and C₁-C₈ alkoxy; and
one of A₁ and A₂ is CH, and the other is N.

2. The compound for use according to Claim 1, wherein R₁ and R₂ are each independently selected from the group consisting of hydrogen, halogen and C₁-C₈ alkyl.

3. The compound for use according to Claim 1 or 2, wherein the carbamate compound of Formula 1 is carbamic acid (R)-1-(2-chlorophenyl)-2-tetrazol-2-yl-ethyl ester of the following Formula 2:

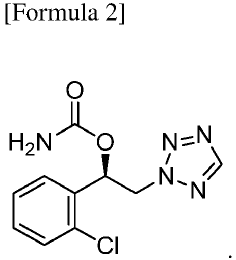

4. The compound for use according to any one of Claims 1 to 3, wherein a subject suffering from the absence seizure or the epilepsy showing absence seizure exhibits spike-and-wave discharges (SWD) in electroencephalography (EEG).

5. The compound for use according to any one of Claims 1 to 4, wherein the epilepsy showing absence seizures is selected from the group consisting of child absence epilepsy, epilepsy with myoclonic absence, juvenile absence epilepsy, juvenile myoclonic epilepsy, Jeavons syndrome (eyelid myoclonia with absence), genetic generalized epilepsy with phantom absence and Lennox-Gastaut syndrome.

6. The compound for use according to any one of Claims 1 to 4, wherein the absence seizure is a typical absence seizure or an atypical absence seizure.

7. The compound for use according to any one of Claims 1 to 6, which is to be used for mammalian administration.

8. The compound for use according to any one of Claims 1 to 7, wherein the amount of the carbamate compound of Formula 1 is 50 mg to 500 mg based on the free form for once-daily administration.

9. A pharmaceutical composition for use in the prevention, alleviation or treatment of absence seizure or epilepsy showing absence seizure, comprising a carbamate compound of the following Formula 1, or a pharmaceutically acceptable salt, solvate or hydrate thereof; and further one or more of a pharmaceutically acceptable carrier: 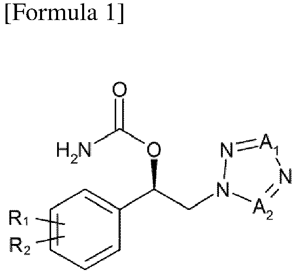 wherein,
R₁ and R₂ are each independently selected from the group consisting of hydrogen, halogen, C₁-C₈ alkyl, halo-C₁-C₈ alkyl, C₁-C₈ thioalkoxy and C₁-C₈ alkoxy; and
one of A₁ and A₂ is CH, and the other is N.

10. The pharmaceutical composition for use according to Claim 9, wherein R₁ and R₂ are each independently selected from the group consisting of hydrogen, halogen and C₁-C₈ alkyl.

11. The pharmaceutical composition for use according to Claim 9 or 10, wherein the carbamate compound of Formula 1 is carbamic acid (R)-1-(2-chlorophenyl)-2-tetrazol-2-yl-ethyl ester of the following Formula 2:

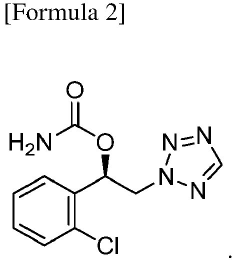

12. The pharmaceutical composition for use according to any one of Claims 9 to 11, wherein a subject suffering from the absence seizure or the epilepsy showing absence seizure exhibits spike-and-wave discharges (SWD) in electroencephalography (EEG).

13. The pharmaceutical composition for use according to any one of Claims 9 to 12, wherein the epilepsy showing absence seizures is selected from the group consisting of child absence epilepsy, epilepsy with myoclonic absence, juvenile absence epilepsy, juvenile myoclonic epilepsy, Jeavons syndrome (eyelid myoclonia with absence), genetic generalized epilepsy with phantom absence and Lennox-Gastaut syndrome.

14. The pharmaceutical composition for use according to any one of Claims 9 to 12, wherein the absence seizure is a typical absence seizure or an atypical absence seizure.

15. The pharmaceutical composition for use according to any one of Claims 9 to 14, which is to be used for mammalian administration.

16. The pharmaceutical composition for use according to any one of Claims 9 to 15, wherein the amount of the carbamate compound of Formula 1 is 50 mg to 500 mg based on the free form for once-daily administration.

## Patentansprüche

1. Eine Carbamatverbindung der folgenden Formel 1 oder ein pharmazeutisch verträgliches Salz, Solvat oder Hydrat davon zur Verwendung bei der Vorbeugung, Linderung oder Behandlung von Absence-Anfällen oder Epilepsie mit Absence-Anfällen: 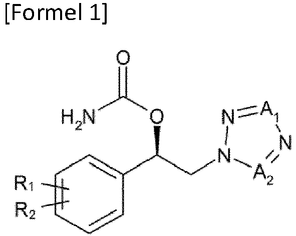 wobei
R₁ und R₂ jeweils unabhängig ausgewählt sind aus der Gruppe bestehend aus Wasserstoff, Halogen, C₁-C₈-Alkyl, Halogen-C₁-C₈-alkyl, C₁-C₈-Thioalkoxy und C₁-C₈-Alkoxy; und
eines von A₁ und A₂ CH ist und das andere N ist.

2. Die Verbindung zur Verwendung nach Anspruch 1, wobei R₁ und R₂ jeweils unabhängig ausgewählt sind aus der Gruppe bestehend aus Wasserstoff, Halogen und C₁-C₈-Alkyl.

3. Die Verbindung zur Verwendung nach Anspruch 1 oder 2, wobei die Carbamatverbindung der Formel 1 Carbaminsäure-(R)-1-(2-chlorphenyl)-2-tetrazol-2-yl-ethylester der folgenden Formel 2 ist:

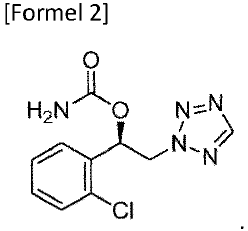

4. Die Verbindung zur Verwendung nach einem der Ansprüche 1 bis 3, wobei eine Person, die an Absence-Anfällen oder Epilepsie mit Absence-Anfällen leidet, Spike-and-Wave-Entladungen (SWD; engl.: spike-and-wave discharges) in der Elektroenzephalographie (EEG) zeigt.

5. Die Verbindung zur Verwendung nach einem der Ansprüche 1 bis 4, wobei die Epilepsie mit Absence-Anfällen ausgewählt ist aus der Gruppe bestehend aus kindlicher Absence-Epilepsie, Epilepsie mit myoklonischer Absence, juveniler Absence-Epilepsie, juveniler myoklonischer Epilepsie, Jeavons-Syndrom (Augenlid-Myoklonie mit Absence), genetisch generalisierter Epilepsie mit Phantom-Absence und Lennox-Gastaut-Syndrom.

6. Die Verbindung zur Verwendung nach einem der Ansprüche 1 bis 4, wobei der Absence-Anfall ein typischer Absence-Anfall oder ein atypischer Absence-Anfall ist.

7. Die Verbindung zur Verwendung nach einem der Ansprüche 1 bis 6, die zur Verabreichung an Säugetiere verwendet werden soll.

8. Die Verbindung zur Verwendung nach einem der Ansprüche 1 bis 7, wobei die Menge der Carbamatverbindung der Formel 1 50 mg bis 500 mg, bezogen auf die freie Form, für einmal tägliche Verabreichung beträgt.

9. Ein Arzneimittel zur Verwendung bei der Vorbeugung, Linderung oder Behandlung von Absence-Anfällen oder Epilepsie mit Absence-Anfällen, umfassend eine Carbamatverbindung der folgenden Formel 1 oder ein pharmazeutisch verträgliches Salz, Solvat oder Hydrat davon; und ferner einen oder mehrere pharmazeutisch verträgliche Träger: 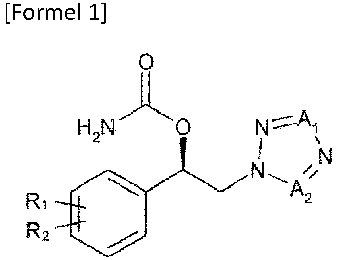 wobei
R₁ und R₂ jeweils unabhängig ausgewählt sind aus der Gruppe bestehend aus Wasserstoff, Halogen, C₁-C₈-Alkyl, Halogen-C₁-C₈-alkyl, C₁-C₈-Thioalkoxy und C₁-C₈-Alkoxy; und
eines von A₁ und A₂ CH ist und das andere N ist.

10. Das Arzneimittel zur Verwendung nach Anspruch 9, wobei R₁ und R₂ jeweils unabhängig ausgewählt sind aus der Gruppe bestehend aus Wasserstoff, Halogen und C₁-C₈-Alkyl.

11. Das Arzneimittel zur Verwendung nach Anspruch 9 oder 10, wobei die Carbamatverbindung der Formel 1 Carbaminsäure-(R)-1-(2-chlorphenyl)-2-tetrazol-2-yl-ethylester der folgenden Formel 2 ist:

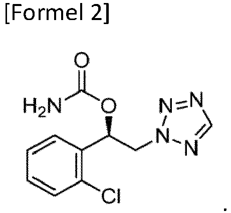

12. Das Arzneimittel zur Verwendung nach einem der Ansprüche 9 bis 11, wobei eine Person, die an Absence-Anfällen oder an Epilepsie mit Absence-Anfällen leidet, Spike-and-Wave-Entladungen (SWD) in der Elektroenzephalographie (EEG) zeigt.

13. Das Arzneimittel zur Verwendung nach einem der Ansprüche 9 bis 12, wobei die Epilepsie mit Absence-Anfällen ausgewählt ist aus der Gruppe bestehend aus kindlicher Absence-Epilepsie, Epilepsie mit myoklonischer Absence, juveniler Absence-Epilepsie, juveniler myoklonischer Epilepsie, Jeavons-Syndrom (Augenlid-Myoklonie mit Absence), genetisch generalisierter Epilepsie mit Phantom-Absence und Lennox-Gastaut-Syndrom.

14. Das Arzneimittel zur Verwendung nach einem der Ansprüche 9 bis 12, wobei der Absence-Anfall ein typischer Absence-Anfall oder ein atypischer Absence-Anfall ist.

15. Das Arzneimittel zur Verwendung nach einem der Ansprüche 9 bis 14, das zur Verabreichung an Säugetiere verwendet werden soll.

16. Das Arzneimittel zur Verwendung nach einem der Ansprüche 9 bis 15, wobei die Menge der Carbamatverbindung der Formel 1 50 mg bis 500 mg, bezogen auf die freie Form, für einmal tägliche Verabreichung beträgt.

## Revendications

1. Composé de carbamate de la formule 1 suivante, ou sel, solvate ou hydrate pharmaceutiquement acceptable de celui-ci pour une utilisation dans la prévention, l'atténuation ou le traitement de crise d'absence ou d'épilepsie avec crise d'absence : 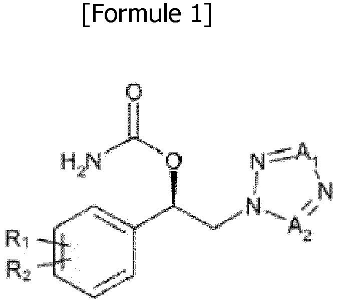 dans laquelle,
R₁ et R₂ sont chacun indépendamment choisis dans le groupe consistant en hydrogène, halogène, alkyle en C₁-C₈, halo-alkyle en C₁-C₈, thioalcoxy en C₁-C₈ et alcoxy en C₁-C₈ ; et
un de A₁ et A₂ est CH, et l'autre est N.

2. Composé pour une utilisation selon la revendication 1, dans lequel R₁ et R₂ sont chacun indépendamment choisis dans le groupe consistant en hydrogène, halogène et alkyle en C₁-C₈.

3. Composé pour une utilisation selon la revendication 1 ou 2, dans lequel le composé de carbamate de formule 1 est le carbamate de (R)-1-(2-chlorophényl)-2-tétrazol-2-yl-éthyle de la formule 2 suivante :

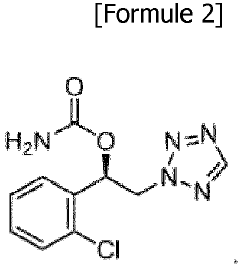

4. Composé pour une utilisation selon l'une quelconque des revendications 1 à 3, dans lequel un sujet souffrant de la crise d'absence ou de l'épilepsie avec absence présente des décharges pointe-et-onde (SWD) dans une électroencéphalographie (EEG).

5. Composé pour une utilisation selon l'une quelconque des revendications 1 à 4, dans lequel l'épilepsie présentant des crises d'absence est choisie dans le groupe consistant en épilepsie avec absence chez l'enfant, épilepsie avec absence myoclonique, épilepsie avec absence juvénile, épilepsie myoclonique juvénile, syndrome de Jeavons (myoclonie de la paupière avec absence), épilepsie généralisée génétique avec absence fantôme et syndrome de Lennox-Gastaut.

6. Composé pour une utilisation selon l'une quelconque des revendications 1 à 4, dans lequel la crise d'absence est une crise d'absence typique ou une crise d'absence atypique.

7. Composé pour une utilisation selon l'une quelconque des revendications 1 à 6, laquelle est destinée à être utilisée pour une administration à un mammifère.

8. Composé pour une utilisation selon l'une quelconque des revendications 1 à 7, dans lequel la quantité du composé de carbamate de formule 1 est de 50 mg à 500 mg sur la base de la forme libre pour une administration une fois par jour.

9. Composition pharmaceutique pour une utilisation dans la prévention, l'atténuation ou le traitement de crise d'absence ou d'épilepsie avec crise d'absence, comprenant un composé de carbamate de la formule 1 suivante, ou un sel, solvate ou hydrate de celui-ci pharmaceutiquement acceptable ; et de plus un ou plusieurs d'un support pharmaceutiquement acceptable : 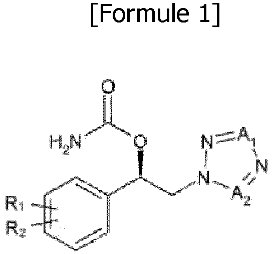 dans laquelle,
R₁ et R₂ sont chacun indépendamment choisis dans le groupe consistant en hydrogène, halogène, alkyle en C₁-C₈, halo-alkyle en C₁-C₈, thioalcoxy en C₁-C₈ et alcoxy en C₁-C₈ ; et
un de A₁ et A₂ est CH, et l'autre est N.

10. Composition pharmaceutique pour une utilisation selon la revendication 9, dans laquelle R₁ et R₂ sont chacun indépendamment choisis dans le groupe consistant en hydrogène, halogène et alkyle en C₁-C₈.

11. Composition pharmaceutique pour une utilisation selon la revendication 9 ou 10, dans laquelle le composé de carbamate de formule 1 est le carbamate de (R)-1-(2-chlorophényl)-2-tétrazol-2-yl-éthyle de la formule 2 suivante :

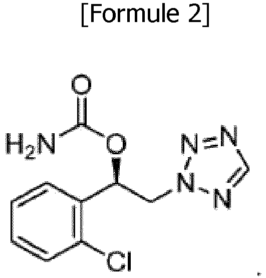

12. Composition pharmaceutique pour une utilisation selon l'une quelconque des revendications 9 à 11, dans laquelle un sujet souffrant de crise d'absence ou de l'épilepsie avec crise d'absence présente des décharges pointe-et-onde (SWD) dans une électroencéphalographie (EEG).

13. Composition pharmaceutique pour une utilisation selon l'une quelconque des revendications 9 à 12, dans laquelle l'épilepsie avec crises d'absence est choisie dans le groupe consistant en épilepsie avec absence chez l'enfant, épilepsie avec absence myoclonique, épilepsie avec absence juvénile, épilepsie myoclonique juvénile, syndrome de Jeavons (myoclonie de la paupière avec absence), épilepsie généralisée génétique avec absence fantôme et syndrome de Lennox-Gastaut.

14. Composition pharmaceutique pour une utilisation selon l'une quelconque des revendications 9 à 12, dans laquelle la crise d'absence est une crise d'absence typique ou une crise d'absence atypique.

15. Composition pharmaceutique pour une utilisation selon l'une quelconque des revendications 9 à 14, laquelle est destinée à être utilisée pour une administration à un mammifère.

16. Composition pharmaceutique pour une utilisation selon l'une quelconque des revendications 9 à 15, dans laquelle la quantité de composé de carbamate de formule 1 est de 50 mg à 500 mg sur la base de la forme libre pour une administration une fois par jour.
